# EUROPÄISCHE PATENTSCHRIFT

(11) **EP 2 995 332 B1**
(45) Veröffentlichungstag und Bekanntmachung des Hinweises auf die Patenterteilung: **09.05.2018**
(21) Anmeldenummer: 15184690.4
(22) Anmeldetag: 10.09.2015
(51) Int. Cl.: A61M 5/158, A61M 5/32, A61M 25/06

(54) **SICHERHEITSVORRICHTUNG FÜR EINE MEDIZINISCHE NADEL**
SAFETY DEVICE FOR A MEDICAL NEEDLE
DISPOSITIF DE SECURITE POUR UNE AIGUILLE MEDICALE

(30) Priorität: 12.09.2014 DE 202014104338 U
(43) Veröffentlichungstag der Anmeldung: 16.03.2016
(73) Patentinhaber: OMT GmbH & Co. KG, 78665 Frittingen (DE)
(72) Erfinder: Mantsch, Christian, 32427 Minden (DE); Stumpp, Uwe, 78665 Frittlingen (DE)
(74) Vertreter: Ter Meer Steinmeister & Partner

(56) Entgegenhaltungen:
- EP-A1- 2 609 953
- WO-A1-2010/101573
- WO-A2-2009/046560
- US-A1- 2005 049 553
- US-A1- 2006 064 061

## Beschreibung

Die Erfindung betrifft eine Sicherheitsvorrichtung zum Abschirmen einer medizinischen Nadel, mit einem Griffelement, einer an daran gehaltenen Kanüle, und einer Basis mit seitlich abstehenden, flachen Flügeln, wobei die Unterseite der Basis im wesentlichen flach ist, und wobei die Basis eine Führungsöffnung aufweist, in der die Kanüle geführt ist, sowie mit einer faltbaren Verbindungsstruktur, die das Griffelement mit der Basis verbindet.

EP 1 682 202 B1 beschreibt eine Abschirmvorrichtung für eine medizinische Nadel, mit einer Abschirmung, die von einer eingeschobenen Position in eine herausgezogene Position ausziehbar ist, um ein distales Ende einer Nadel zu umschließen. Die Vorrichtung umfasst ein Außenlager und ein separates Innenlager, das in einem Innenraum des Außenlagers entlang der Längsachse bewegbar ist. Ein gelenkiges, aus mehreren Segmenten aufgebautes Halteseil verbindet das Außenlager mit einer Nabe, an der eine Kanüle in Form einer Huber-Nadel montiert ist. Ein mit dem Innenlager bewegbarer Verkeilungsabschnitt enthält eine Nockenfläche, die in eine Seitenwand des Außenlagers eingreift, um den Verkeilungsabschnitt zu drehen und gegen die Nadel zu verkeilen, wenn das Innenlager innerhalb des Außenlagers nach oben gezogen wird. Das Verschwenken des Verkeilungsabschnitts erfolgt um eine Schwenkachse, die quer zu den Gelenkachsen von Scharnieren des Halteseils angeordnet ist. Das Halteseil verhindert, dass in der herausgezogenen Position der Nadel die Abschirmung von der Nadel getrennt wird.

EP 2 206 528 A1, EP 2 332 594 A2, US 6,997,902 B2, US 7,347,842 B2, US 7,351,230 B2 und US 7,758,544 B2 sowie US 2006/0064061 A1 beschreiben ähnliche Abschirmvorrichtungen.

EP 2609953 A1 beschreibt eine Sicherheitsvorrichtung für medizinische Nadeln, mit einem Festhalteelement und einem Schildelement, welches eine Verbindungsnabe und Beinabschnitte umfasst. Eine Nadel wird in einen Raum der Verbindungsnabe eingesetzt, wenn die Beinabschnitte in einer ersten, gefalteten Position sind. Wenn die Beinabschnitte durch Hochziehen von Flügeln in eine zweite, ausgestreckte Position gebracht werden, wird die Verbindungsnabe in dem Festhalteelement nach oben gezogen, und ein Gleitelement wird verschwenkt und blockiert den Querschnitt des Raums, wodurch das distale Ende der Nadel im Inneren der Vorrichtung geschützt ist.

US 2005/0049553 A1 beschreibt eine Sicherheitsvorrichtung mit zwei Hauptteilen, einer Basis und einer Nadel. Die Hauptteile sind über einen Arm mit einer Auflage verbunden. Ein gelenkiges, faltbares Element begrenzt eine Aufwärtsbewegung der Hauptteile gegenüber der Auflage beim Herausziehen der Nadel aus einem Port. Beim Hindurchtreten der seitlich versetzten Nadelspitze durch eine distale Gehäuseöffnung schnappt die Nadelspitze hinter einen Gehäusevorsprung zurück.

WO 2010/101573 A1 betrifft eine medizinische Vorrichtung mit einer Nadel und einem Nadelgehäuse, in welchem die Nadelspitze verriegelt werden kann.

WO 2009/046560 A2 betrifft eine Sicherheitsanordnung für eine Kanüle mit einem verschiebbaren Schutzelement.

Aufgabe der Erfindung ist es, eine Sicherheitsvorrichtung für eine medizinische Portnadel zu schaffen, die sich dadurch auszeichnet, dass sie die Nadel nach Gebrauch besonders gut gegen einen Zugriff abschirmt.

Diese Aufgabe wird erfindungsgemäß gelöst durch eine Sicherheitsvorrichtung nach Anspruch 1.

Durch Festhalten der Basis an den Flügeln und Hochziehen des Griffelements wird die Verbindungsstruktur aufgefaltet, während gleichzeitig die Kanüle zusammen mit dem Griffelement aufwärts gezogen wird. Indem beim Auffalten der Verbindungsstruktur der mit der Basis mittels des wenigstens eines Gelenks verbundene untere längliche Abschnitt der Verbindungsstruktur aufgerichtet wird, wird dessen quer abstehendes Wandelement zusammen mit dem Abschnitt verschwenkt. D.h. das Wandelement, das mit dem unteren Abschnitt unmittelbar gekoppelt ist und vorzugsweise fest mit dem unteren Abschnitt verbunden ist, wird zusammen mit diesem verschwenkt, so dass bereits durch das Auffalten der Verbindungsstruktur das Wandelement seine Endposition einnimmt, in der es sich vor der Spitze der Kanüle befindet. Dadurch ist sichergestellt, dass in dem ausgestreckten Zustand der Verbindungsstruktur sich das Wandelement quer vor der Spitze der Kanüle befindet. Dies entspricht dem gesicherten Zustand der Sicherheitsvorrichtung, in dem die Spitze der Kanüle zwischen der Führungsöffnung und dem Wandelement angeordnet und so gegen einen Zugriff abgeschirmt ist. Das Auffalten der Verbindungsstruktur in den ausgestreckten Zustand bringt somit automatisch die Sicherheitsvorrichtung in den gesicherten Zustand. Besonders vorteilhaft ist, dass eine sehr geringe Anzahl beweglicher Teile dennoch eine sichere Abschirmung der Kanülenspitze ermöglicht. So kann das quer abstehende Wandelement beispielsweise einstückig mit dem länglichen Abschnitt der Verbindungsstruktur geformt sein. Indem das quer abstehende Wandelement und der längliche Abschnitt der Verbindungsstruktur, an dem es angeordnet ist, gemeinsam verschwenkt werden, ergibt sich eine hohe Funktionssicherheit der Abschirmung.

Bevorzugte Ausgestaltungen der Erfindung ergeben sich aus den Unteransprüchen.

Vorzugsweise umfassen die Abschnitte der Verbindungsstruktur jeweils zwei Teile, insbesondere zwei Teile in Form von Abschirmwänden, die beidseitig der Kanüle sich gegenüberliegend und neben dieser in Längsrichtung der Kanüle verlaufend angeordnet sind, wenn die Verbindungsstruktur in dem zweiten Zustand linear ausgestreckt ist. Die beidseitig der Kanüle angeordneten Teile schützen die Kanüle gegen seitliche Berührung.

Vorzugsweise weisen die Teile Abschirmwände auf, die sich mit ihren flachen Seiten gegenüberliegen. Dadurch kann die Kanüle besonders gut gegen Berührung abgeschirmt werden. Denn die Abschirmwände, die in Längsrichtung des jeweiligen Abschnitts verlaufen, schirmen einen zwischen ihnen aufgespannten Raum gegen Berührung ab.

Vorzugsweise ist ein erster der länglichen Abschnitte der Verbindungsstruktur an einem Ende mit dem Griffelement und am anderen Ende mit dem zweiten der länglichen Abschnitte jeweils über wenigstens ein Gelenk in Form eines Folienscharniers verbunden. Dadurch kann der Aufbau der Verbindungsstruktur vereinfacht werden.

Vorzugsweise weist der mit der Basis mittels wenigstens eines Gelenks verbundene längliche Abschnitt der Verbindungsstruktur Achszapfen auf, die in Aufnahmen der Basis gelagert sind, wobei die Gelenkzapfen und die Aufnahmen das wenigstens ein Gelenk bilden. Dies ermöglicht, die Verbindungsstruktur und die Basis aus unterschiedlich harten Materialien herzustellen. Außerdem kann so ein besonders stabiles Gelenk gebildet werden.

Vorzugsweise sind das Griffelement, die länglichen Abschnitte der Verbindungsstruktur, das Wandelement und die Achszapfen zusammen einstückig aus Kunststoff geformt. Dadurch wird ein vereinfachter und besonders kompakter Aufbau ermöglicht.

Vorzugsweise ist der mit der Basis mittels wenigstens eines Gelenks verbundene längliche Abschnitt der Verbindungsstruktur in seiner Längsrichtung geteilt, wobei die beiden Teile am Basis-seitigen Ende des Abschnitts über das quer abstehende Wandelement miteinander fest verbunden sind, und wobei der Abschnitt auf einander gegenüberliegenden Seiten Achszapfen aufweist, die die Gelenkachse des Gelenks bilden, wobei, in Richtung auf die Gelenkachse gesehen, die beiden Teile mit dem Wandabschnitt eine L-förmige Anordnung bilden. Vorzugsweise verläuft die Gelenkachse durch den Winkel der L-Form. Dadurch wird die Schwenkbewegung des Abschnitts beim Auffalten der Verbindungsstruktur besonders effizient in eine Schwenkbewegung des Wandelements in seine Stellung vor der Spitze der Kanüle umgesetzt.

Vorzugsweise ist die Verbindungsstruktur entlang ihre Längsrichtung geteilt, und die Teilung erstreckt sich über die Verbindung der beiden länglichen Abschnitte, so dass die Verbindungsstruktur in jeden der Abschnitte zwei Teile aufweist, wobei die Abschnitte durch ein Gelenk in Form von zwei Folienscharnieren verbunden sind, die jeweils einen Teil des einen Abschnitts mit einem Teil des anderen Abschnitts verbinden, und wobei die Teile eines jeweiligen Abschnitts sich gegenüberliegend beidseitig der Kanüle und neben dieser in Längsrichtung der Kanüle verlaufend angeordnet sind, wenn die Verbindungsstruktur in dem zweiten Zustand linear ausgestreckt ist. Durch diesen geteilten Aufbau der Verbindungsstruktur kann die Verbindungsstruktur an der Kanüle vorbei aufgefaltet werden.

Vorzugsweise weist die Basis wenigstens ein Sperrelement auf, das, wenn die Verbindungsstruktur in dem zweiten Zustand linear ausgestreckt ist, hinter dem mit der Basis mittels eines Gelenks verbundenen Abschnitt der Verbindungsstruktur angeordnet ist, so dass dieser Abschnitt gegen ein Zurückfalten gesperrt ist. Das Sperrelement kann beispielsweise ein Keil sein, über den der Abschnitt der Verbindungsstruktur unter elastischer Verformung des Keils und/oder des Abschnitts hinweg bewegbar ist, wenn die Verbindungsstruktur von dem ersten in den zweiten Zustand aufgefaltet wird. Alternativ oder zusätzlich zu dem Sperrelement weist die Basis vorzugsweise einen Rastvorsprung auf, der, wenn die Verbindungsstruktur in dem zweiten Zustand linear ausgestreckt ist, unter dem Wandelement des mit der Basis mittels eines Gelenks verbundenen Abschnitts der Verbindungsstruktur angeordnet ist, so dass dieser Abschnitt gegen ein Zurückfalten gesperrt ist. Durch das Sperrelement und/oder den Rastvorsprung kann die Verbindungsstruktur auf einfache Weise wirksam vor dem erneuten Zusammenfalten geschützt werden.

Ein bevorzugtes Ausführungsbeispiel der Erfindung wird im folgenden anhand der Zeichnung näher erläutert.

Es zeigen:
- Fig. 1: eine schematische Seitenansicht in teilweiser Schnittdarstellung einer erfindungsgemäßen Sicherheitsvorrichtung in einem gesicherten Zustand;
- Fig. 2: eine perspektivische, schematische Darstellung eines unteren Endes einer Verbindungsstruktur der Sicherheitsvorrichtung;
- Fig. 3: eine Seitenansicht auf einen Teil der Sicherheitsvorrichtung;
- Fig. 4: die Sicherheitsvorrichtung in einem verwendungsbereiten Zustand einer Portnadel in einer Ansicht entsprechend Fig. 1;
- Fig. 5: eine schematische Querschnittsansicht eines Teils einer Basis der Sicherheitsvorrichtung;
- Fig. 6: eine perspektivische Ansicht der Sicherheitsvorrichtung in dem verwendungsbereiten Zustand gemäß Fig. 4; und
- Fig. 7: eine perspektivische Ansicht der Sicherheitsvorrichtung im gesicherten Zustand gemäß Fig. 1 und 3.

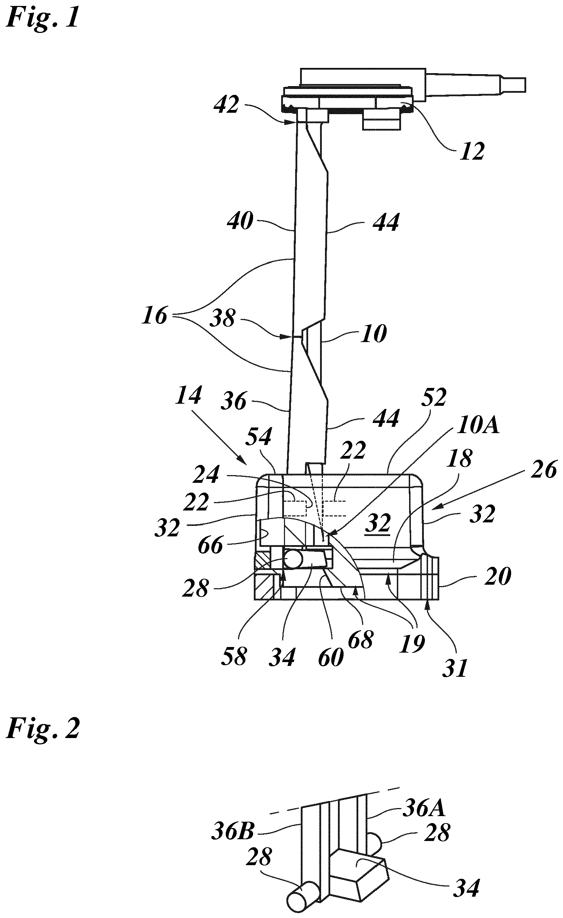

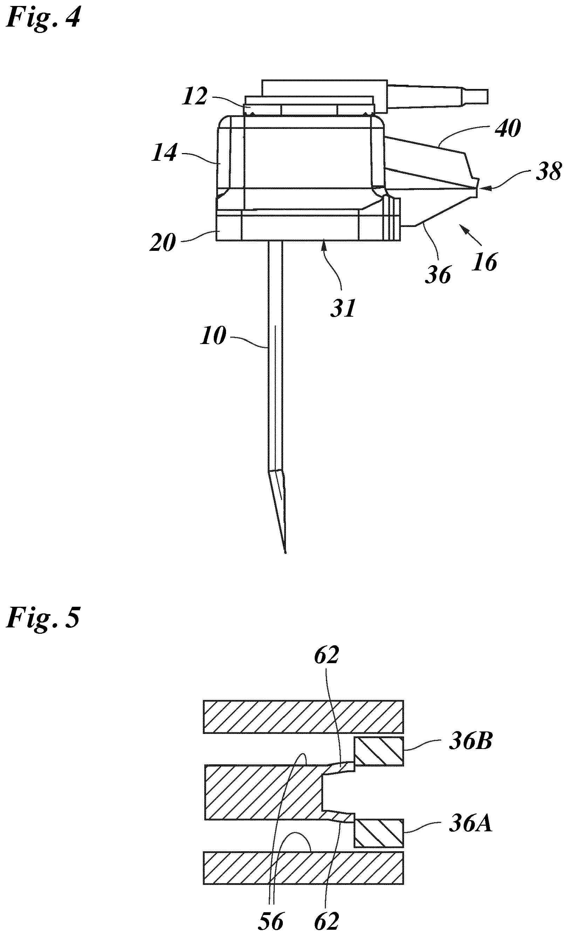

Fig. 1 zeigt eine erfindungsgemäße Sicherheitsvorrichtung zum Abschirmen einer medizinischen Nadel, insbesondere einer Portnadel 10, mit einem Griffelement 12, einer Basis 14 und einer Verbindungsstruktur 16, die das Griffelement 12 mit der Basis 14 verbindet. Bei der Portnadel 10 handelt es sich um eine Hohlnadel oder Kanüle, die am Griffelement 12 gehalten ist und einen im wesentlichen geraden Abschnitt aufweist, der am Griffelement 12 ansetzt und an einer Spitze 10A der Portnadel 10 endet. Die Portnadel 10 ist insbesondere eine Hubernadel, die im Bereich der Kanülenspitze 10A leicht abgewinkelt ist, wie Fig. 4 zeigt. Die Basis 14 ist in Fig. 1 in einem Teilbereich im Schnitt dargestellt, wobei dort die Verbindungsstruktur 16 nicht im Schnitt, sondern voll dargestellt ist.

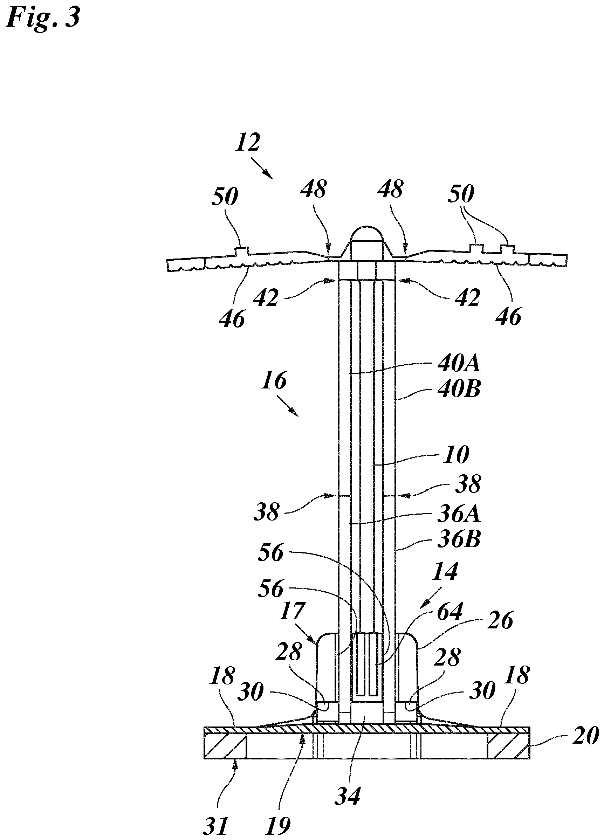

Wie Fig. 3 zeigt, hat die Basis einen Grundkörper 17 und davon seitlich abstehende, flache Flügel 18, die am unteren Rand der Basis 14 angeordnet sind. Die sich über die Flügel 18 und den mittleren Grundkörper 17 der Basis erstreckende Unterseite 19 der Basis 14 ist im wesentlichen flach und mit einem Auflagekissen 20 aus Schaumstoff verbunden, z.B. verklebt, das flach an der Unterseite 19 der Basis 14 anliegt und eine im wesentlichen ringförmige, breite Stützfläche zum Abstützen der Basis 14 auf der Haut eines Patienten bildet. Das Auflagekissen 20 hat beispielsweise eine Stärke von 1 bis 2 mm.

Beabstandet von der Unterseite 19 der Basis 14 ist in einer oberen Wand 22 eine Führungsöffnung 24 für die Kanüle ausgespart, in die die Kanüle 10 eintaucht. Die Kanüle 10 ist unmittelbar, d.h. ohne dazwischenliegende Bauteile, in der Führungsöffnung 24 mit etwas Spiel oder, vorzugsweise, ohne Spiel geführt.

Die Basis hat im wesentlichen die Form einer Platte, von der ein oberseitiger, kastenförmiger Anbau 26 aufragt, wobei die Platte die an den beiden gegenüberliegenden Seiten des Anbaus 26 vorstehenden Flügel 18 bildet. Der Grundkörper 17 umfasst den Anbau 26.

Die Verbindungsstruktur 16 taucht mit ihrem unterseitigen Ende in den Anbau 26 der Basis 14 ein und ist dort mit beidseitigen Achszapfen 28 in Zapfenaufnahmen 30 der Basis 14 gelagert. Die Achszapfen 28 bilden eine Gelenkachse eines Drehgelenks. Die Gelenkachse verläuft in Fig. 1 senkrecht zur Zeichenebene und verläuft parallel zur Auflagefläche 31 des Auflagekissens 20 an der Unterseite 19 der Basis 14. Die Achszapfen 28, und somit die Gelenkachse, ist senkrechter Richtung zur Unterseite 19 der Basis 14 von der Führungsöffnung 24 beabstandet.

Der zwischen der Führungsöffnung 24 und der Höhe der Achszapfen 28 liegende Innenraum der Basis 14, in dem in Fig. 1 die Spitze 10A der Kanüle 10 angeordnet ist, ist an vier Seiten durch Seitenwände 32 des Anbaus 26 umgeben und ist nach oben durch die obere Wand 22 begrenzt. Dieser Innenraum ist in der in Fig. 1 gezeigten, linear ausgestreckten Stellung der Verbindungsstruktur 16 durch ein Wandelement 34 nach unten verschlossen, so dass die Spitze 10A der Kanüle 10 an sechs Seiten, d.h. umlaufend sowie oben und unten, abgeschirmt ist. Das Wandelement 34 ist Teil der Verbindungsstruktur 16 und zwischen den Achszapfen 28 angeordnet und fest mit diesem verbunden. In der Stellung in Fig. 1 ragt das Wandelement 34 von der Gelenkachse des Drehgelenks 28, 30 seitlich und im wesentlichen horizontal vor.

Oberhalb der Achszapfen 28 und im rechten Winkel zum Wandelement 34 ragen zwei parallel zueinander angeordnete Teile 36A, 36B eines unteren Abschnitts 36 der Verbindungsstruktur 16 auf. Diese Teile 36A, 36B sind an ihren oberen Enden über Drehgelenke in Form von Folienscharnieren 38 jeweils mit einem von zwei parallel zueinander angeordneten Teilen 40A, 40B eines oberen Abschnitts 40 der Verbindungsstruktur 16 verbunden. Die Teile 40A, 40B sind wiederum an ihren oberen Enden jeweils über ein Drehgelenk in Form eines Folienscharniers 42 mit dem Griffelement 12 verbunden.

Die Teile 36A, 36B, 40A, 40B der Verbindungsstruktur sind jeweils längliche, steife Elemente aus Kunststoff und weisen jeweils eine Abschirmwand 44 auf, wobei sich in jedem der unteren und oberen Abschnitte 36, 40 der Verbindungsstruktur 16 die Abschirmwände 44 mit ihren flachen Seiten gegenüberstehen. In der in Fig. 1 gezeigten Stellung ist die Kanüle 10 zwischen den sich gegenüberliegenden Abschirmwänden 44 angeordnet und verläuft entlang der Längsrichtung der Teile 36A, 36B, 40A, 40B. Die Kanüle 10 ist dadurch unterhalb des Griffelements 12 von den Teilen und Abschirmwänden 44 gegen einen Zugriff abgeschirmt. Insbesondere ist der nach der Anwendung der Portnadel aus der Basis 14 herausgezogene Schaft der Nadel durch seine Anordnung innerhalb des von den beidseitig angeordneten Abschirmwänden 44 aufgespannten, schlauch- oder kanalförmigen Raumes gut gegen einen Zugriff abgeschirmt. Die Abschirmwände 44 bilden somit eine längliche Abschirmung, die sich über den wesentlichen Teil der Länge der Verbindungsstruktur 16 erstreckt.

In der in Fig. 1 dargestellten Blickrichtung auf die Gelenkachse des Drehgelenks 28, 30 bildet das Wandelement 34 mit dem Abschnitt 36 eine umgekehrt L-förmige Anordnung oder eine L-förmige Anordnung. Wie in Fig. 2 zu sehen ist, verläuft die Gelenkachse durch den Winkel der L-Form.

Fig. 3 zeigt die Anordnung der Kanüle 10 zwischen den linear ausgestreckten Abschnitten der Teile 36A, 36B und 40A, 40B.

Das Griffelement 12 weist an seinen gegenüberliegenden Seiten abstehende Flügel 46 auf, die über Folienscharniere 48 mit einem mittleren Abschnitt des Griffelements 12 verbunden sind und in an sich bekannter Weise nach oben gegeneinander gefaltet werden können. Im gegeneinander gefaltetem Zustand sind die Flügel 46 über Rastelemente 50 miteinander verbindbar.

Das Griffelement 12 mit den Flügeln 46 sowie die Verbindungsstruktur 16 mit den Abschnitten der Teile 36 und 40 einschließlich des vom unteren Abschnitt quer abstehenden Abschnitts 34 und den Achszapfen 28 ist einstückig aus Kunststoff als Spritzteil hergestellt, vorzugsweise aus einem relativ flexiblen, weichen Kunststoff wie Polyvinylchlorid (PVC).

Wie in Fig. 1 gezeigt ist, umfasst der Anbau 26 der Basis 14 einen Hauptteil 52 und eine Endkappe 54. Der Hauptteil 52 weist Schlitze für die Teile 36A, 36B auf. Die Endkappe 54 ist beispielsweise über ein Folienscharnier 58 mit dem Hauptteil 52 verbunden.

Die Schlitze 56 sowie die Zapfenaufnahmen 30 liegen bei geöffneter Endkappe 54 frei. Fig. 3 zeigt entspricht einer Blickrichtung in Fig. 1 von links, wobei die Endkappe 54 weggelassen ist. Zur Montage wird bei offener Endkappe 54 die mit der Portnadel 10 montierte Verbindungsstruktur 16 mit den Achszapfen 28 in die offenliegenden Zapfenaufnahmen 30 und die Schlitze 56 eingesetzt. Fig. 3 zeigt schematisch Rastelemente 64 der Endkappe 54, die in eine Rastaufnahme 66 (Fig. 1) des Hauptteils 52 einrastbar ist. Durch Schließen und Verrasten der Endkappe 54 werden die Zapfenaufnahmen 30 geschlossen, so dass die Position der Gelenkachse der Achszapfen 28 festgelegt ist.

Die Basis 14 mit den Flügeln 18 und dem Anbau 26 ist beispielsweise einstückig mit der Endkappe 54 aus einem relativ harten, beispielsweise transparenten Kunststoff hergestellt, beispielsweise aus Polyoxymethylen (POM), Polycarbonat, oder transparentem Acrylnitril-Butadien-Styrol (ABS). Dabei ist es besonders vorteilhaft, dass durch den einfachen Aufbau des Drehgelenks 28, 30, mit dem die Verbindungsstruktur 16 einschließlich des Wandelements 34 an der Basis 14 gelagert ist, die Basis 14 mit der über das Folienscharnier verbundene Endkappe 54 einstückig hergestellt werden kann.

Die drei Gelenke an den Enden sowie in der Mitte in der Verbindungsstruktur 16 sind Drehgelenke, d. h. sie gestatten eine Gelenkbewegung um genau eine Gelenkachse. Wie in Fig. 1, 3 und 4 zu sehen ist, ist die Gelenkachse des Drehgelenks 28, 30 parallel zu den Gelenkachsen der Folienscharniere 38 und 42. Die Abschnitte 36 ,40 der Verbindungsstruktur 16 bilden somit eine Beinstruktur mit zwei Beinen mit jeweils einem Kniegelenk in Form des Folienscharniers 38, einem unteren Schenkel in Form eines Teils 36A, 36B und einen oberen Schenkel in Form eines Teils 40A, 40B.

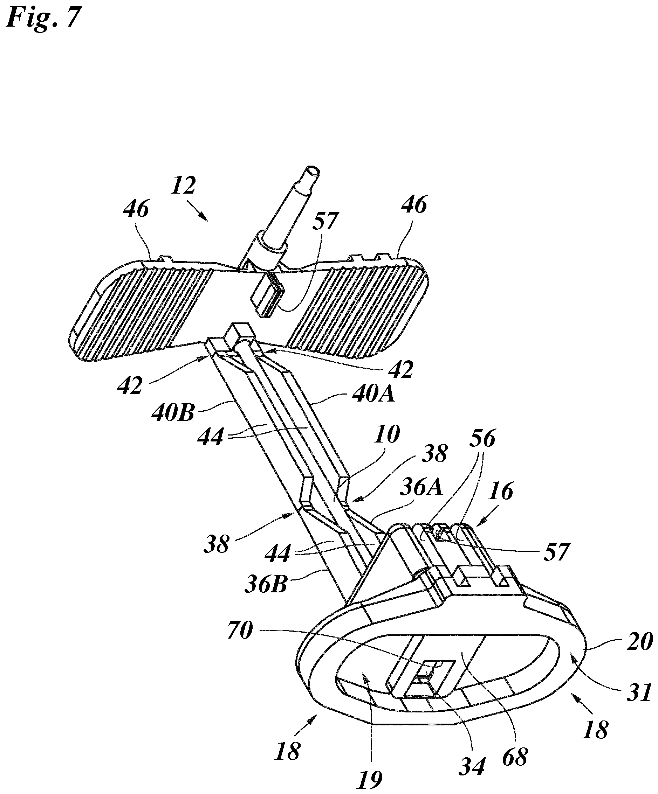

Fig. 4 zeigt einen ersten, zusammengefalteten Zustand der Verbindungsstruktur 16, bei dem die Kanüle 10 mit dem überwiegenden Teil ihrer Länge unterhalb der Unterseite 19 der Basis 14 vertikal vorsteht. In dieser einsatzbereiten Stellung der Portnadel sind die Teile 36A, 36B, 40A, 40B mit den Abschirmwänden 44 in den sich über die Länge des Hauptteils 52 erstreckenden Schlitzen 56 aufgenommen und ragen an der der Endkappe 54 gegenüberliegenden Seite aus der Basis 14 aus deren Seitenwand 32 vor. Das Griffelement 12 nimmt mit den Abschnitten 40 und 36 eine im wesentlichen Z-förmig zusammengefaltete Stellung ein, wobei das Griffelement 12 an der Basis 14 anliegt und beispielsweise mit Rastelementen 57 (Fig. 7) daran verrastet oder gehalten ist. In der zusammengefalteten Stellung kreuzen die Teile 36A, 36B die Kanüle 10, welche durch den Zwischenraum zwischen den Teilen 36A, 36B verläuft. Dadurch kann das Wandelement 34 von unten vor den Innenraum der Basis geschwenkt werden, bis es in Stellung der Fig. 1 vor der Spitze 10A der Kanüle ist. Auch die Teile 40A, 40B kreuzen in der Stellung der Fig. 4 die Kanüle 10.

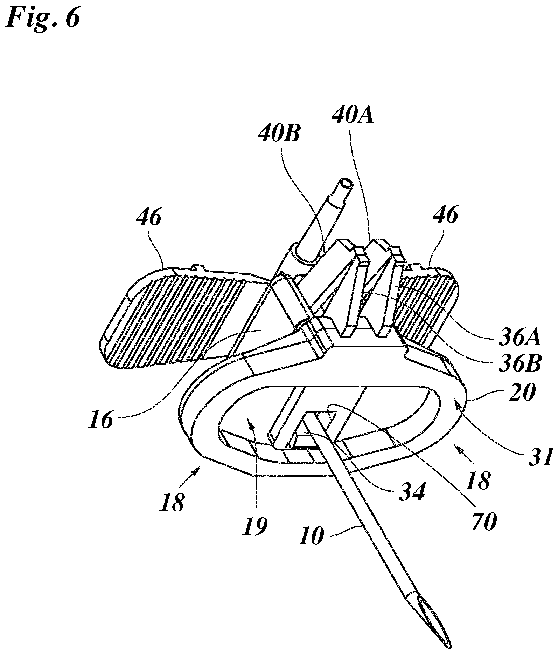

In der in Fig. 4 und 6 gezeigten Stellung, die geeignet zum Einführen der Kanüle 10 in einen implantierten Port geeignet ist, kann die Vorrichtung flach auf der Haut des Patienten befestigt werden. Das Wandelement 34 befindet sich neben dem Schaft der Kanüle 10, wie Fig. 6 zeigt. Nach Anwendung der Portnadel können die Flügel 46 nach oben geklappt werden, und durch Ergreifen des Griffelements 12 an den zusammengeklappten Flügeln 46 wird dieses nach oben gezogen, während die Basis 14 durch oberseitiges Auflegen zweier Finger auf die Flügel 18 gehalten wird. In der Aufwärtsbewegung wird die Kanüle 10 in der Führungsöffnung 24 geführt, und die Verbindungsstruktur 16 wird aus dem zusammengefalteten Zustand in den linear ausgestreckten Zustand der Fig. 1 aufgefaltet. Dabei schwenkt synchron mit der Schwenkbewegung der Teile 36A, 36B das Wandelement 34 aus der vertikal nach unten gerichteten Stellung in die in Fig. 1 gezeigte, im wesentlichen horizontale Stellung bezüglich der Auflagefläche 31 der Basis 14. Die Kanüle 10 gleitet beispielsweise am Wandelement 34 entlang, bis schließlich in der Endstellung die Spitze 10A der Kanüle 10 oberhalb des Wandelements 34 angeordnet ist und z.B. in Richtung der Fläche des Wandelements 34 gerichtet ist.

Während des Auffaltens der Verbindungsstruktur 16 und dem Bewegen des Wandelements 34 in die Endstellung vor der Spitze 10A der Kanüle 10 hält der Grundkörper 17 der Basis 14 das Gelenk 28, 30 stets in festem Abstand und vorzugsweise in fester Position zur Oberseite der Flügel 18 am seitlichen Ansatz der Flügel 18 an dem Grundkörper 17, und somit auch in festem Abstand zur Unterseite 19 der Basis 14. Es erfolgt somit keine Aufwärtsbewegung der Gelenkachse des Gelenks 28, 30, sondern allein eine Drehung. Insbesondere ist der Basis-seitige Gelenkteil des Gelenks 28, 30, d.h. die Zapfenaufhahmen 30, fest verbunden und vorzugsweise einstückig geformt mit dem seitlichen Ansatz der Flügel 18 an dem Grundkörper 17 der Basis 14. Das Gelenk 28, 30 ist somit unverrückbar verbunden mit dem Ansatz der Flügel 18 am Grundkörper 17. Dadurch kann eine definierte Auffaltbewegung der Verbindungsstruktur 16 durch Festhalten der Flügel 18 und Hochziehen der Kanüle 10 mittels des Griffelements 12 erfolgen. Beim Auffalten der Verbindungsstruktur und Hochziehen der Kanüle bis in die gesicherte Stellung der Sicherheitsvorrichtung wird somit allein die Verbindungsstruktur teilweise angehoben, jedoch kein die Kanüle umringender Teil der Basis 14. Insbesondere bleibt die Führungsöffnung 24 der Wand 22 stets in festem Abstand und in fester Position zur Oberseite der Flügel 18 am seitlichen Ansatz der Flügel 18, und somit auch in festem Abstand zur Unterseite 19 der Basis 14.

Optional weist die Basis 14 einen zweigeteilten Rastvorsprung 60 auf, an dem vorbei unter elastischer Verformung des Rastvorsprungs 60 und/oder des Wandelements 34 das Wandelement in die Endstellung geschwenkt wird, wobei in der Endstellung die Rastvorrichtung 60 vor der Unterseite des Wandelements 34 vorsteht und so das Wandelement 34 in der Abschirmstellung verrastet und sichert, wie Fig. 1 zeigt.

Wie in Fig. 5 schematisch dargestellt ist, weist die Basis 14 optional Sperrelemente 62 auf, die rampenförmig in die Schlitze 56 hineinragen. Bei der Auffaltbewegung der Verbindungsstruktur 16 laufen die Teile 36A, 36B unter elastischer Verformung der Sperrelemente 62 und/oder einer elastischen Ausweichbewegung der Teile 36A, 36B über die Sperrelemente 62 hinweg. In der in Fig. 1 und Fig. 5 gezeigten Endstellung der Verbindungsstruktur 16 sind die Sperrelemente 62 dann vor den Teilen 36A, 36B so angeordnet, dass diese gegen ein Zurückschwenken gesperrt sind.

Beim Überwinden des Rastvorsprungs 60 durch das Wandelement 34 und/oder beim Überwinden der Sperrelemente 62 durch die Teile 36 wird ein hörbares Klickgeräusch erzeugt, um einem Anwender der Sicherheitsvorrichtung das Erreichen des sicheren, verriegelten Endzustands anzuzeigen.

In der Stellung in Fig. 1 wird durch die gesicherte Anordnung der Kanülenspitze 10A innerhalb der Basis 14 und die Anordnung des Schaftes der Kanüle 10 zwischen den Abschirmwänden 44 ein besonders guter Schutz vor versehentlicher Berührung der gebrauchten Kanüle 10 und insbesondere der Spitze 10A geschaffen. Die Position der Spitze 10A ist dabei durch die Länge der Verbindungsstruktur 16 in vertikaler Richtung festgelegt, während in den Richtungen quer zur vertikalen Richtung bezogen auf die flache Unterseite 19 der Basis 14, die Position der Spitze 10A der Kanüle 10 durch die Kanüle umgebende Führungsöffnung 24 festgelegt ist. Die Kanüle 10 ist dabei weiterhin am Griffelement 12 gehalten.

Fig. 6 zeigt schematisch in perspektivischer Ansicht von schräg unten die Unterseite 19 der Basis 14 mit dem ringförmigen Auflagekissen 20, das die vorstehende Kanüle 10 umgibt, in der anwendungsbereiten Stellung der Portnadel 10 gemäß Fig. 4.

Fig. 7 zeigt schematisch in perspektivischer Darstellung die Sicherheitsvorrichtung mit linear ausgestreckter Verbindungsstruktur 16. Die Spitze 10A der Kanüle 10 ist vollständig in die Basis 14 hinein hinter das Wandelement 34 gezogen.

Wie Fig. 7 zeigt, weist die Basis 14 im Bereich um die unterseitige Öffnung einen plattenförmigen, den die unterseitige Öffnung umgebenden Flächenabschnitt 68 auf, der etwas tiefer liegt als die umgebende Fläche der Unterseite 19 der Basis 14. Der Flächenabschnitt 68 ragt beispielsweise bis zur halben Stärke des Auflagekissens 20 nach unten vor, so dass die Unterseite 19 der Basis 14 dennoch im wesentlichen flach ist.

Fig. 6 und 7 zeigen weiter eine Öffnung 70 im Boden der Basis 14 im Bereich des Flächenabschnitts 68, durch die bei zusammengefalteter Verbindungsstruktur 16 die Kanüle 10 hindurchgeht, und in welcher das Wandelement 34 aus seiner nach unten gerichteten Stellung in die abschirmende Stellung schwenkbar ist.

## Patentansprüche

1. Sicherheitsvorrichtung zum Abschirmen einer medizinischen Nadel, aufweisend:
ein Griffelement (12);
eine Kanüle (10), die an dem Griffelement (12) gehalten ist;
eine Basis (14) mit seitlich abstehenden, flachen Flügeln (18), wobei die Unterseite (19) der Basis (14), die sich über die Flügel (18) erstreckt, im wesentlichen flach ist, und wobei die Basis (14) eine von der Unterseite (19) beabstandete Führungsöffnung (24) aufweist, in der die Kanüle (10) geführt ist; und
eine Verbindungsstruktur (16), die das Griffelement (12) mit der Basis (14) verbindet und zwei längliche, in einer Reihe miteinander mittels wenigstens eines Gelenks (38) verbundene Abschnitte (36; 40) umfasst, wobei die Verbindungsstruktur (16) an einem Ende mittels wenigstens eines Gelenks (42) mit dem Griffelement (12) verbunden ist und an dem anderen Ende mittels wenigstens eines Gelenks (28, 30) mit der Basis (14) verbunden ist,
wobei der mit der Basis (14) mittels des wenigstens einen Gelenks (28, 30) verbundene längliche Abschnitt (36) der Verbindungsstruktur (16) an seinem Basis-seitigen Ende ein quer abstehendes Wandelement (34) aufweist, und
wobei die Abschnitte (36; 40) und die Gelenke (28, 30; 38; 42) so angeordnet sind, dass die Verbindungsstruktur (16) aus einem ersten Zustand, in welchem die in der Führungsöffnung (24) der Basis (14) geführte Kanüle (10) aus der Unterseite (19) der Basis (14) mit der Spitze (10A) vorsteht, in einen zweiten, ausgestreckten Zustand auffaltbar ist, in welchem die Kanüle (10) längs der Verbindungsstruktur (16) angeordnet ist, das Wandelement (34) quer vor der Spitze (10A) der Kanüle (10) angeordnet ist, und eine Seitenwand (32) der Basis (14) die Spitze (10A) zwischen der Führungsöffnung (24) und dem Wandelement (34) schützend umgibt und gegen einen Zugriff abschirmt.

2. Sicherheitsvorrichtung nach Anspruch 1, wobei wenigstens einer der Abschnitte (36; 40) der Verbindungsstruktur (16) jeweils zwei Teile (36A, 36B; 40A, 40B) umfasst, die sich gegenüberliegend beidseitig der Kanüle (10) und neben dieser in Längsrichtung der Kanüle (10) verlaufend angeordnet sind, wenn die Verbindungsstruktur (16) in dem zweiten Zustand linear ausgestreckt ist.

3. Sicherheitsvorrichtung nach Anspruch 2, wobei die Teile (36A, 36B; 40A, 40B) Abschirmwände (44) aufweisen, die sich mit ihren flachen Seiten gegenüberliegen.

4. Sicherheitsvorrichtung nach Anspruch 3, wobei, wenn die Verbindungsstruktur (16) in dem zweiten Zustand linear ausgestreckt ist, ein Schaft der Kanüle (10) zwischen den sich gegenüberliegenden Abschirmwänden (44) angeordnet ist und entlang der Längsrichtung der Teile (36A, 36B; 40A, 40B) verläuft.

5. Sicherheitsvorrichtung nach einem der Ansprüche 1 bis 4, bei der der mit der Basis (14) mittels wenigstens eines Gelenks (28, 30) verbundene längliche Abschnitt (36) der Verbindungsstruktur (16) zwei Teile (36A, 36B) umfasst, die im ersten Zustand der Verbindungsstruktur (16) die Kanüle (10) kreuzen, wobei die Kanüle (10) durch einen Zwischenraum zwischen den nebeneinander und beabstandet voneinander angeordneten Teilen (36A; 36B) hindurchgeht.

6. Sicherheitsvorrichtung nach einem der Ansprüche 1 bis 5, bei der das wenigstens eine Gelenk (38), welches die zwei länglichen Abschnitte (36; 40) verbindet, und die Gelenke (28, 30; 42), mittels welchen die Verbindungsstruktur (16) an ihren Enden mit dem Griffelement (12) und der Basis (14) verbunden sind, jeweilige Gelenkachsen aufweisen, welche parallel zueinander sind.

7. Sicherheitsvorrichtung nach einem der Ansprüche 1 bis 6, bei der ein erster Abschnitt (40) der länglichen Abschnitte (36; 40) der Verbindungsstruktur (16) an einem Ende mit dem Griffelement (12) und am anderen Ende mit dem zweiten Abschnitt (36) der länglichen Abschnitte (36; 40) jeweils über wenigstens ein Gelenk in Form eines Folienscharniers (38; 42) verbunden ist.

8. Sicherheitsvorrichtung nach einem der Ansprüche 1 bis 7, bei der der mit der Basis (14) mittels wenigstens eines Gelenks (28, 30) verbundene längliche Abschnitt (36) der Verbindungsstruktur (16) Achszapfen (28) aufweist, die in Aufnahmen (30) der Basis (14) gelagert sind, wobei die Achszapfen (28) und die Aufnahmen (30) das wenigstens eine Gelenk (28, 30) bilden.

9. Sicherheitsvorrichtung nach Anspruch 8, bei der das Griffelement (12), die länglichen Abschnitte (36; 40) der Verbindungsstruktur (16), das Wandelement (34) und die Achszapfen (28) zusammen einstückig aus Kunststoff geformt sind.

10. Sicherheitsvorrichtung nach einem der Ansprüche 1 bis 9, bei der der mit der Basis (14) mittels wenigstens eines Gelenks (28, 30) verbundene längliche Abschnitt (36) der Verbindungsstruktur (16) in seiner Längsrichtung geteilt ist, die beiden Teile (36) am Basis-seitigen Ende des Abschnitts (36) über das quer abstehende Wandelement (34) miteinander fest verbunden sind, und der Abschnitt (36) auf einander gegenüberliegenden Seiten Achszapfen (28) aufweist, die eine Gelenkachse des Gelenks (28, 30) bilden, wobei, in Richtung auf die Gelenkachse gesehen, die beiden Teile (36) mit dem Wandabschnitt (34) eine L-förmige Anordnung bilden.

11. Sicherheitsvorrichtung nach Anspruch 10, wobei die Gelenkachse durch den Winkel der L-Form verläuft.

12. Sicherheitsvorrichtung nach einem der Ansprüche 1 bis 11, bei der die Flügel (18) der Basis (14) an einem Grundkörper (17) der Basis (14) seitlich ansetzen, wobei der Grundkörper (17) der Basis das wenigstens eine Gelenk (28, 30), mittels welchem die Verbindungsstruktur (16) an dem anderen Ende mit der Basis (14) verbunden ist, in einem festen Abstand zur Oberseite der Flügel (18) am seitlichen Ansatz der Flügel (18) an dem Grundkörper (17) der Basis (14) hält.

13. Sicherheitsvorrichtung nach einem der Ansprüche 1 bis 11, bei der die Verbindungsstruktur (16) entlang ihrer Längsrichtung geteilt ist und die Teilung sich über die Verbindung der beiden länglichen Abschnitte (36; 40) erstreckt, so dass die Verbindungsstruktur (16) in jedem der Abschnitte (36; 40) zwei Teile (36A, 36B; 40A, 40B) aufweist, und wobei die Abschnitte (36; 40) durch ein Gelenk in Form von zwei Folienscharnieren (38) verbunden sind, die jeweils einen Teil (36A; 36B) des einen Abschnitts (36) mit einem Teil (40A; 40B) des anderen Abschnitts (40) verbinden, und wobei die Teile (36A, 36B; 40A, 40B) eines jeweiligen Abschnitts (36; 40) sich gegenüberliegend beidseitig der Kanüle (10) neben dieser in Längsrichtung der Kanüle (10) verlaufend angeordnet sind, wenn die Verbindungsstruktur (16) in dem zweiten Zustand linear ausgestreckt ist.

## Claims

1. A safety device for shielding a medical needle, comprising:
a hand grip element (12);
a cannula (10), which is held at the hand grip element (12);
a base (14) with laterally protruding, flat wings (18), wherein the underside (19) of the base (14), which extends over the wings (18), is essentially flat and wherein the base (14) has a guide opening (24) which is spaced apart from the underside (19) and in which the cannula (10) is guided; and
a connecting structure (16) which connects the grip element (12) with the base (14) and comprises two elongated sections (36,40), connected in a row with one another by at least one joint (38), wherein the connecting structure (16) is connected at one end by at least one joint (42) with the hand grip element (12) and, at an opposite end, by at least one joint (28, 30) with the base (14),
wherein the elongated section (36) of the connecting structure (16) which is connected with the base (14) by the at least one joint (28, 30), has a wall element (34) which protrudes transversely at a base end thereof, and
wherein the sections (36, 40) and the joints (28, 30; 38; 42) are disposed so that the connecting structure (16) can be unfolded from a first state, in which the cannula (10), which is guided in the guide opening (24) of the base (14), protrudes with the tip (10A) thereof from the underside (19) of the base (14), into a second extended state, in which the cannula (10) is disposed along the connecting structure (16), the wall element (34) is disposed transversely in front of the tip (10A) of the cannula (10), and a side wall (32) of the base (14) protectively surrounds the tip (10A) between the guide opening (24) and the wall element (34) and shields the tip (10A) against access.

2. The safety device of claim 1, wherein at least one of the sections (36; 40) of the connecting structure (16) each comprises two parts (36A, 36B; 40A, 40B) which are disposed mutually opposite to one another on either side of the cannula (10) and extend next to the cannula (10) in a longitudinal direction of the cannula (10), when the connecting structure (16) is extended linearly in the second state.

3. The safety device of claim 2, wherein the parts (36A, 36B; 40A, 40B) have shielding walls (44), which, with flat sides thereof, lie opposite to one another.

4. The safety device of claim 3, wherein, when the connecting structure (16) is extended linearly in the second state, a shaft of the cannula (10) is disposed between the mutually opposite shielding walls (44) and extends along the longitudinal direction of the parts (36A, 36B; 40A, 40B).

5. The safety device according to any one of claims 1 to 4, wherein the elongated section (36) of the connecting structure (16) which is connected with the base (14) by at least one joint (28, 30), comprises two parts (36A, 36B) which cross the cannula (10) in the first state of the connecting structure (16), the cannula (10) passing through a space between the parts (36A; 36B), which are disposed next to one another and spaced apart.

6. The safety device according to any one of claims 1 to 5, wherein the at least one joint (38) which connects the two elongated sections (36; 40), and the joints (28, 30; 42) by which the ends of the connecting structure (16) are connected with the hand grip element (12) and the base (14), have respective joint axes, which are parallel to one another.

7. The safety device according to any one of claims 1 to 6, wherein a first elongated section (40) of the elongated sections (36; 40) of the connecting structure (16) is connected at one end with the hand grip element (12), and, at an opposite end, with the second section (36) of the elongated sections (36,40) in each case over at least one joint in the form of a living hinge (38; 42).

8. The safety device according to any one of claims 1 to 7, wherein the elongated section (36) of the connecting structure (16) which is connected with the base (14) by the at least one joint (28, 30), has kingpins (28), which are mounted in receivers (30) of the base (14), the kingpins (28) and the receivers (30) forming the at least one joint (28, 30).

9. The safety device of claim 8, wherein the hand grip element (12), the elongated sections (36; 40) of the connecting structure (16), the wall element (34) and the kingpins (28) are formed together in one piece from a plastic material.

10. The safety device according to any one of claims 1 to 9, wherein the elongated section (36) of the connecting structure (16) which is connected with the base (14) by the at least one joint (28, 30), is divided in a longitudinal direction thereof, the two parts (36) being firmly connected with one another at a base-side end of the section (36) over the transversely protruding wall element (34), and said section (36), on mutually opposite sides, has kingpins (28) which form an axis of the joint (28, 30), and the two parts (36), as seen in a direction of the joint axis, forming an L-shaped arrangement with the wall section (34).

11. The safety device of claim 10, wherein the joint axis passes through the angle of the L-shape.

12. The safety device according to any one of claims 1 to 11, wherein the wings (18) of the base (14) are mounted laterally at a main body (17) of the base (14), and wherein the main body (17) of the base holds the at least one joint (28, 30) by which the connecting structure (16) is connected at the other end with the base (14), at a fixed distance to the upper side of the wings (18) at the lateral root of the wings (18) at the main body (17) of the base (14).

13. The safety device according to any one of claims 1 to 11, wherein the connecting structure (16) is divided along a longitudinal direction thereof and the division thereof extends over the connection of the two elongated sections (36; 40), so that the connecting structure (16) has two parts (36A, 36B; 40A, 40B) in each of the sections (36; 40) and wherein the sections (36; 40) are connected by a joint in the form of two living hinges (38), each of which connects one part (36A; 36B) of one section (36) with one part (40A; 40B) of the other section (40), and wherein the parts (36A, 36B; 40A, 40B) of each section (36; 40) are disposed so that they extend mutually opposite to one another on either side of the cannula (10) next to the cannula (10) in a longitudinal direction of the cannula (10), when the connecting structure (16) is extended linearly in the second state.

## Revendications

1. Dispositif de sécurité destiné à protéger une aiguille médicale, comportant :
un élément de préhension (12),
une canule (10) qui est maintenue sur l'élément de préhension (12),
une base (14) munie d'ailes plates (18) faisant latéralement saillie, dans lequel la face inférieure (19) de la base (14) qui s'étend au-dessus des ailes (18) est sensiblement plane, et dans lequel la base (14) comporte une ouverture de guidage (24) espacée de la face inférieure (19), dans laquelle la canule (10) est guidée, et
une structure de liaison (16) qui relie l'élément de préhension (12) à la base (14) et qui comprend deux portions longitudinales (36 ; 40) reliées l'une à l'autre au moyen d'au moins un joint articulé (38) en formant une ligne, dans lequel la structure de liaison (16) est reliée, au niveau d'une extrémité, à l'élément de préhension (12) au moyen d'au moins un joint articulé (42) et est reliée, au niveau de l'autre extrémité, à la base (14) au moyen d'au moins un joint articulé (28, 30),
dans lequel la portion longitudinale (36) de la structure de liaison (16) reliée à la base (14) au moyen du au moins un joint articulé (28, 30) comporte, à son extrémité côté base, un élément de paroi (34) faisant saillie transversalement, et
dans lequel les portions (36 ; 40) et les joints articulés (28, 30 ; 38 ; 42) sont agencées de sorte que la structure de liaison (16) peut être dépliée depuis un premier état, dans lequel la canule (10) guidée dans l'ouverture de guidage (24) de la base (14) fait saillie avec la pointe (10A) à partir de la face inférieure (19) de la base (14), jusqu'à un second état étendu, dans lequel la canule (10) est agencée le long de la structure de liaison (16), l'élément de paroi (34) est agencé transversalement avant la pointe (10A) de la canule (10), et une paroi latérale (32) de la base (14) entoure la pointe (10A) entre l'ouverture de guidage (24) et l'élément de paroi (34) de manière à la protéger et empêche d'y accéder.

2. Dispositif de sécurité selon la revendication 1, dans lequel au moins une des portions (36 ; 40) de la structure de liaison (16) comprend respectivement deux parties (36A, 36B ; 40A, 40B) qui sont agencées de manière mutuellement opposée des deux côtés de la canule (10) et à côté de celle-ci dans une direction longitudinale de la canule (10), lorsque la structure de liaison (16) est étendue linéairement dans le second état.

3. Dispositif de sécurité selon la revendication 2, dans lequel les parties (36A, 36B ; 40A, 40B) comportent des parois de protection (44) qui sont opposées l'une à l'autre avec leurs faces plates.

4. Dispositif de sécurité selon la revendication 3, dans lequel, lorsque la structure de liaison (16) est étendue linéairement dans le second état, une tige de la canule (10) est agencée entre les parois de protection (44) mutuellement opposées et s'étend le long de la direction longitudinale des parties (36A, 36B ; 40A, 40B).

5. Dispositif de sécurité selon l'une des revendications 1 à 4, dans lequel la portion longitudinale (36) de la structure de liaison (16) reliée à la base (14) au moyen d'au moins un joint articulé (28, 30) comprend deux parties (36A, 36B) qui croisent la canule (10) dans le premier état de la structure de liaison (16), dans lequel la canule (10) traverse un espace entre les parties (36A ; 36B) agencées l'une à côté de l'autre et espacées l'une de l'autre.

6. Dispositif de sécurité selon l'une des revendications 1 à 5, dans lequel le au moins un joint articulé (38) qui relie les deux portions longitudinales (36 ; 40), et les joints articulés (28, 30 ; 42) au moyen desquels la structure de liaison (16) est reliée, au niveau de son extrémité, à l'élément de préhension (12) et à la base (14), comportent des axes d'articulation respectifs qui sont parallèles l'un à l'autre.

7. Dispositif de sécurité selon l'une des revendications 1 à 6, dans lequel une première portion (40) des portions longitudinales (36 ; 40) de la structure de liaison (16) est respectivement reliée, au niveau d'une extrémité, à l'élément de préhension (12) et, au niveau de l'autre extrémité, à la seconde portion (36) de la portion longitudinale (36 ; 40) par l'intermédiaire d'au moins un joint articulé ayant la forme d'une charnière à film (38 ; 42).

8. Dispositif de sécurité selon l'une des revendications 1 à 7, dans lequel la portion longitudinale (36) de la structure de liaison (16) reliée à la base (14) au moyen d'au moins un joint articulé (28, 30) comporte des tourillons (28) qui sont montés dans des évidements (30) de la base (14), dans lequel les tourillons (28) et les évidements (30) forment le au moins un joint articulé (28, 30).

9. Dispositif de sécurité selon la revendication 8, dans lequel l'élément de préhension (12), les portions longitudinales (36 ; 40) de la structure de liaison (16), l'élément de paroi (34) et les tourillons (28) sont formés ensemble d'un seul tenant à partir d'une matière plastique.

10. Dispositif de sécurité selon l'une des revendications 1 à 9, dans lequel la portion longitudinale (36) de la structure de liaison (16) reliée à la base (14) au moyen d'au moins un joint articulé (28, 30) est divisée dans sa direction longitudinale, les deux parties (36) sur l'extrémité côté base de la portion (36) sont fixement reliées l'une à l'autre par l'élément de paroi faisant saillie transversalement (34), et la portion (36) comporte, sur des côtés opposés l'un à l'autre, des tourillons (28) qui forment un axe d'articulation du joint articulé (28, 30), dans lequel, lorsque vues en direction de l'axe d'articulation, les deux parties (36) forment un agencement en L avec la portion de paroi (34).

11. Dispositif de sécurité selon la revendication 10, dans lequel l'axe d'articulation s'étend sur l'angle de la forme en L.

12. Dispositif de sécurité selon l'une des revendications 1 à 11, dans lequel les ailes (18) de la base (14) sont montées latéralement sur un corps principal (17) de la base (14), dans lequel le corps de base (17) de la base maintient le au moins un joint articulé (28, 30), au moyen duquel la structure de liaison (16) est reliée à la base (14) à l'autre extrémité, à une distance fixe par rapport à la face supérieure de l'aile (18) sur un appendice latéral de l'aile (18) sur le corps principal (17) de la base (14).

13. Dispositif de sécurité selon l'une des revendications 1 à 11, dans lequel la structure de liaison (16) est divisée le long de sa direction longitudinale et la division s'étend sur la liaison des deux portions longitudinales (36 ; 40), de sorte que la structure de liaison (16) comporte deux parties (36A, 36B ; 40A, 40B) dans chacune des portions (36 ; 40), et dans lequel les portions (36 ; 40) sont reliées par un joint articulé ayant la forme de deux charnières à film (38) qui relient respectivement une partie (36A ; 36B) de la première portion (36) à une partie (40A ; 40B) de l'autre portion (40), et dans lequel les parties (36A, 36B ; 40A, 40B) d'une portion (36 ; 40) respective sont agencées de manière opposée l'une à l'autre des deux côtés de la canule (10) et à côté de celle-ci dans la direction longitudinale de la canule (10), lorsque la structure de liaison (16) est étendue linéairement dans le second état.
